# EUROPEAN PATENT SPECIFICATION

(11) **EP 2 095 115 B1**
(45) Date of publication and mention of the grant of the patent: **16.05.2012**
(21) Application number: 07815137.0
(22) Date of filing: 03.10.2007
(51) Int. Cl.: G01N 33/574

(54) **GENERAL PROGNOSTIC PARAMETERS FOR TUMOUR PATIENTS**
ALLGEMEINE PROGNOSEPARAMETER FÜR TUMORPATIENTEN
PARAMÈTRES DE PRONOSTIC GÉNÉRAL POUR DES PATIENTS ATTEINTS D'UNE TUMEUR

(30) Priority: 03.10.2006 AT 16522006
(43) Date of publication of application: 02.09.2009
(73) Proprietor: Stoiber, Wolfgang, 82031 Grünwald (DE); Loibner, Hans, 1230 Vienna (AT)
(72) Inventor: LOIBNER, Hans, A-1230 Vienna (AT)
(74) Representative: Sonn & Partner Patentanwälte
(86) International application number: PCT/AT2007/000470
(87) International publication number: WO 2008/040045

(56) References cited:
- LUC F E MERCELINA ET AL: "Leucocyte Count and Leucocyte Differential in Patients with Early Stage Breast Cancer" SYSMEX JOURNAL INTERNATIONAL, TOA MEDICAL LECTRONICS CO., KOBE, JP, vol. 8, 1998, pages 38-40, XP001537675 ISSN: 0917-7205
- WALSH S R ET AL: "Neutrophil-lymphocyte ratio as a prognostic factor in colorectal cancer." JOURNAL OF SURGICAL ONCOLOGY 1 SEP 2005, vol. 91, no. 3, 1 September 2005 (2005-09-01), pages 181-184, XP002460743 ISSN: 0022-4790
- VENTAFRIDDA V ET AL: "Leucocyte-lymphocyte ratio as prognostic indicator of survival in cachectic cancer patients." ANNALS OF ONCOLOGY : OFFICIAL JOURNAL OF THE EUROPEAN SOCIETY FOR MEDICAL ONCOLOGY / ESMO MAR 1991, vol. 2, no. 3, March 1991 (1991-03), page 196, XP009092924 ISSN: 0923-7534
- PAPATESTAS A E ET AL: "The prognostic significance of peripheral lymphocyte counts in patients with breast carcinoma" CANCER 1976, vol. 37, no. 1, 1976, pages 164-168, XP009092923 ISSN: 0008-543X
- KIM U-S ET AL: "PROGNOSTIC SIGNIFICANCE OF PERIPHERAL LYMPHOCYTE COUNTS AND CARCINO EMBRYONIC ANTIGENS IN COLO RECTAL CARCINOMA" JOURNAL OF SURGICAL ONCOLOGY, vol. 8, no. 3, 1976, pages 257-262, XP009092921 ISSN: 0022-4790
- FUMAGALLI LUCA A ET AL: "Lymphocyte counts independently predict overall survival in advanced cancer patients: A biomarker for IL-2 immunotherapy." JOURNAL OF IMMUNOTHERAPY, vol. 26, no. 5, September 2003 (2003-09), pages 394-402, XP009092899 ISSN: 1524-9557 cited in the application
- SCHNEIDER SANDRA: "Lymphocyte counts influence the prognosis" ZEITSCHRIFT FUER GASTROENTEROLOGIE, vol. 44, no. 7, July 2006 (2006-07), page 568, XP009092933 ISSN: 0044-2771
- ECKSCHLAGER T: "LYMPHOCYTE COUNT AS A PROGNOSTIC FACTOR IN CHILDHOOD CANCER" PEDIATRIC HEMATOLOGY AND ONCOLOGY, vol. 9, no. 2, 1992, pages 99-105, XP009092934 ISSN: 0888-0018
- ATZPODIEN J ET AL: "Metastatic renal carcinoma comprehensive prognostic system." BRITISH JOURNAL OF CANCER, vol. 88, no. 3, 10 February 2003 (2003-02-10), pages 348-353, XP002460745 ISSN: 0007-0920 cited in the application
- SCHMIDT H ET AL: "Elevated neutrophil and monocyte counts in peripheral blood are associated with poor survival in patients with metastatic melanoma: a prognostic model" BRITISH JOURNAL OF CANCER, vol. 93, no. 3, August 2005 (2005-08), pages 273-278, XP002460746 ISSN: 0007-0920 cited in the application
- DONSKOV F ET AL: "Monocytes and neutrophils as 'bad guys' for the outcome of interleukin-2 with and without histamine in metastatic renal cell carcinoma - results from a randomised phase II trial" BRITISH JOURNAL OF CANCER, vol. 94, no. 2, January 2006 (2006-01), pages 218-226, XP002460747 ISSN: 0007-0920 cited in the application
- ZAHOREC R: "Ratio of neutrophil to lymphocyte counts--rapid and simple parameter of systemic inflammation and stress in critically ill." BRATISLAVSKÉ LEKÁRSKE LISTY 2001, vol. 102, no. 1, 2001, pages 5-14, XP002460748 ISSN: 0006-9248 cited in the application

## Description

The present invention relates to the determination of parameters which allow a prognosis of clinical benefit of a tumor patient.

There is increasing evidence that the immune system already plays a critical role in controlling early events in the recognition and destruction of first malignant cells, but importantly a proper function is also very important in later stages of the disease to control and stabilise progression (at least for a certain time). It is, however, well known that the immune system of cancer patients is often impaired due to the disease and also due to immunosuppressive therapies. Cancer immunotherapies, such as therapies involving cancer vaccines, rely on a proper function of the immune system as one of the key requisites for a possible clinical efficacy.

A white cell blood count enumerating various types of white blood cells as one parameter of the status of the immune system is always measured in the course of a cancer therapy. Thus, the number of neutrophils or the number of lymphocytes is always determined in tumor patients undergoing clinical trials. These data have been correlated by some groups with the success of the cancer therapy and prognosis in a few cancer indications. See, for example Walsh et al (J. of surgical oncology. 2005, 91: 181-184). For example, Fumagalli et al. (J Immunother. 2003 Sep-Oct; 26(5): 394-402) reported that lymphocyte counts independently predicted overall survival in advanced cancer patients as a biomarker for IL-2 immunotherapy. In this study it was retrospectively evaluated whether lymphocytosis, in addition to clinical characteristics at baseline and to tumor objective response, might predict overall survival in metastatic renal cell carcinoma patients who received IL-2 subcutaneously (s.c.). Overall survival, clinical characteristics, tumor response, and total lymphocyte count at baseline and during the first treatment cycle of 266 advanced renal cell cancer patients, being treated with 1 of 4 different first-line s.c. IL-2-based protocols, were studied using the Cox multivariate analysis. A two-step bootstrapping procedure confirmed such predictive performance. Lymphocyte count monitoring was regarded to represent a biomarker of the host response to subcutaneous IL-2 treatment useful for multimodal clinical assessment, as it predicts overall survival in advanced cancer patients independently from tumor response and from main clinical characteristics.

Ownby et al. (Cancer. 1983 Jul 1;52(1):126-30) have published that peripheral lymphocyte and eosinophil counts could be used as indicators of prognosis in primary breast cancer. As a part of a major study on the pathophysiologic indices for recurrence of human breast cancer, preoperative eosinophil and lymphocyte counts were determined on 419 and 581 primary breast cancer patients, respectively. Patients with lymphocyte counts less than or equal to 1500/mm3 and/or eosinophil counts of less than 55/mm3 had significantly higher risk of recurrent disease than those patients who had normal or high levels of eosinophils and/or lymphocytes. These findings were reported to possibly indicate that the immunologic activities of eosinophils and lymphocytes enhance the patients' ability to respond against disease.

The purpose of the study of Atzpodien et al. (Br J Cancer. 2003 Feb 10;88(3):348-53) was to identify a comprehensive prognostic system of pretreatment clinical parameters in 425 patients (pts) with metastatic renal-cell carcinoma treated with different subcutaneous (s.c.) recombinant cytokine-based home therapies in consecutive trials. Treatment consisted of (A) s.c. interferon-alpha 2a (INF-alpha), s.c. interleukin-2 (IL-2) (n=102 pts), (B) s.c. IFN-alpha 2a, s.c. IL-2, and i.v. 5-fluorouracil (5-FU) (n=235 pts) or (C) s.c. IFN-alpha 2a, s.c. IL-2, and i.v. 5-FU combined with p.o. 13-cis-retinoic acid (13cRA) (n=88 pts). Kaplan-Meier survival analysis, log-rank statistics, and Cox regression analysis were employed to identify risk factors and to create a multiple risk factor model. The following pretreatment risk factors were identified by univariate analysis: (1) three and more metastatic sites, (2) presence of liver, lymph node or bone metastases, (3) neutrophil count > or = 6500 cells microl (-1), (4) serum lactate dehydrogenase level (LDH) > or = 220 U 1(-1), and (5) serum C-reactive protein level (CRP) > or = 11 mg 1(-1). Cox regression analysis with forward stepwise variable selection identified neutrophil counts as the major prognostic factor (hazard ratio = 1.9, P<0.001), while serum levels of LDH and CRP, time between diagnosis of tumor and onset of metastatic disease, number of metastatic sites, and bone metastases were significant but somewhat less important prognostic variables within the multiple risk factor model (hazard ratio < or = 1.5).

The total and differential leucocyte counts were determined by Luc et al. (Sysmex J Int 8 : 38-40, 1998) in 81 patients with newly diagnosed early stage breast cancer, classified as clinical stage 0, I or II and compared with the same counts in 39 age matched healthy women and 67 patients with a benign breast lesion. None of the patients selected for this study had any other medical disorder that could influence these counts. Significantly lower lymphocyte, basophil and eosinophil counts were found in the patients with early stage breast cancer, when compared with the healthy age matched control group. The neutrophil counts were significantly higher in patients with a benign or malignant lesion of the breast in comparison with the control group. There were no significant alterations in the total leucocyte counts in the three groups.

Zahorec (Bratisl Lek Listy 2001; 102 (1): 5.14) investigated the ratio of neutrophil to lymphocyte counts as a rapid and simple parameter of systemic inflammation and stress in critically ill. Rapid serial changes in white blood cell populations were observed in 90 ICU oncological patients as a response of the immune system to surgical stress, systemic inflammation or sepsis. Preliminary results showed a correlation between the severity of clinical course and the grade of neutrophilia and lymphocytopenia. The ratio of neutrophil and lymphocyte counts (in absolute and/or relative % values) was suggested to be an easily measurable parameter which may express the severity of affliction. The ratio of neutrophil to lymphocyte counts (neutrophil-lymphocyte stress factor) was suggested to be routinely used in clinical ICU practice in intervals of 6-12 and 24 hours. The prognostic value of neutrophil-lymphocyte stress factor was proposed to be evaluated in further studies.

Paesmans et al. (J Clin Oncol. 1995 May;13(5):1221-30 and Cancer. 2000 Aug 1;89(3):523-33) described prognostic factors for survival in advanced non-small-cell lung cancer. It was attempted to determine the prognostic value for survival of various pretreatment characteristics in patients with nonresectable non-small-cell lung cancer in the context of more than 10 years of experience of a European Cooperative Group. 23 variables were prospectively collected and analysed by univariate and multivariate methods. However, the long term prognosis associated with small cell lung carcinoma is poor. The well-known prognostic values of disease extent and Karnofsky performance status were confirmed, but the authors also identified age and gender (which are more controversial) as independent characteristics, in addition to citing the role of complete response in the attainment of long term survival. The independent role of neutrophils observed by the authors was reported as having to be validated by further studies.

It is becoming clearer and clearer that inflammation plays a crucial role in cancer development. Inflammation is a critical component of tumor progression. Many cancers arise from sites of infection, chronic irritation and inflammation. It is now becoming clear that the tumor microenvironment, which is largely orchestrated by inflammatory cells, is an indispensable participant in the neoplastic process, fostering proliferation, survival and migration. In addition, tumor cells have co-opted some of the signalling molecules of the innate immune system, such as selectins, chemokines and their receptors for invasion, migration and metastasis. These insights are fostering new anti-inflammatory therapeutic approaches to cancer development (Coussens et al. (Nature 240 (2002))).

McMillan et al. (Nutr Cancer. 2001;41(1-2):64-9) reported that measurement of the systemic inflammatory response predicted cancer-specific and non-cancer survival in patients with cancer. Patients with a diagnosis of colorectal (n = 182), gastric (n = 87), breast (n = 99), or bronchogenic (n = 404) cancer and who had measurements of C-reactive protein and albumin were identified. Median survival, from the time of sampling, ranged from 478 days in the colorectal cancer patients to 60 days in patients with bronchogenic cancer. On univariate analysis, there was, in each tumor type, a significant relationship between the duration of survival and both log10 C-reactive protein and albumin concentrations (P < or = 0.0002). On multivariate analysis, in each tumor type, log10 C-reactive protein remained a significant independent predictor of survival (P < or = 0.0002). When all four groups of cancer patients were analysed (n = 772), the hazard ratio for a 10-fold increase in C-reactive protein concentration in cancer-specific survival was 2.21 (95% confidence interval = 1.92-2.56, P < 0.0001) and the corresponding hazard ratio for non-cancer survival was 5.48 (95% confidence interval = 3.55-8.46, P < 0.0001). The results of this study indicated that in advanced cancer patients the presence of a systemic inflammatory response and the magnitude of that response predicted the duration of cancer-specific and non-cancer survival.

Nozoe et al. (American Journal of Clinical Oncology. 23(3): 263-266, June 2000) reported the significance of a preoperative elevation of serum C-reactive protein (CRP) as an indicator of the malignant potential and prognosis in colorectal cancer. The reduction of circulating lymphocytes was said to reflect the immunosuppressive conditions of patients with neoplasms. It was shown that preoperative elevation of serum CRP was significantly related to the reduction of lymphocyte percentages in peripheral blood, and that it could be an indicator of impaired immunity in the patients with colorectal cancer.

Schmidt et al. (Brit.J.Cancer 93 (2005), 273-278) report that elevated neutrophil and monocyte counts in peripheral blood are associated with poor survival in patients with metastatic melanoma receiving IL-2-based immunotherapy.

It is an object of the present invention to provide objective immunological parameters measured at baseline (for clinical trials: preferably before first treatment of enrolled patients) that have a prognostic impact on the overall clinical benefit that a patient might enjoy, especially a prognostic parameter for overall survival (OS). These parameters should be easily detectable and manageable.

Therefore, the present invention provides a method for identifying the prognosis for improved clinical benefit of an individual suffering from a tumor comprising
- providing a blood sample of said individual,
- (a1) determining the number of lymphocytes in the blood of said individual and/or
- (a2) determining the number of neutrophils in the blood of said individual, and
- (b1) identifying the individual as having a good prognosis for improved clinical benefit, if the number of lymphocytes is above a lymphocyte baseline level of 1.4 to 1.8 x 10⁹ per liter blood, especially above 1.6 x 10⁹ per liter blood; or
- (b2) identifying the individual as having a good prognosis for improved clinical benefit, if the number of neutrophils is below or equal to a neutrophil baseline level of from 4.0 to 6.0 x 10⁹ per liter blood, especially below or equal to 5.0 x 10⁹ neutrophils per liter blood; or
- (b3) identifying the individual as having a bad prognosis for improved clinical benefit, if the number of lymphocytes is below or equal to a lymphocyte baseline level of 1.4 to 1.8 x 10⁹ per liter blood, especially below or equal to 1.6 x 10⁹ per liter blood and the number of neutrophils is above a neutrophil baseline level of from 4.0 to 6.0 x 10⁹ per liter blood, especially above 5.0 x 10⁹ neutrophils per liter blood.

According to the present invention, it was surprisingly determined that neutrophil numbers and lymphocyte numbers in individuals could be efficient prognosis markers for clinical benefits of tumor patients, if baselines were considered which are usually within the "normal" values of individuals. It is clear that tumor patients which already have a lymphocyte or neutrophil number within pathological ranges have a bad prognosis in general. However, the present invention makes use of a significant difference in prognosis within the physiological range of neutrophil numbers and lymphocyte numbers or combinations of these numbers (especially the difference of lymphocytes and neutrophils).

Usually, neutrophil numbers within 1.7 and 7.5 x 10⁹ per liter blood, lymphocyte numbers within 1.2 and 4.5 x 10⁹ per liter blood, or leukocyte numbers within 4 and 10 x 10⁹ per liter blood, are regarded as physiological ("normal"). Pathological values differ from source to source, e.g. neutropenia is reported to be associated with less than 1 x 10⁹ neutrophils; severe neutropenia is associated with less than 0.5 x 10⁹ neutrophils. Lymphopenia is associated with less than 1 x 10⁹ lymphocytes per liter blood; leukopenia with less than 4 x 10⁹ leukocytes per liter blood. According to the present invention, individuals with a number of lymphocytes or neutrophils within the physiological range are contemplated, i.e. with at least 0.5 x 10⁹ lymphocytes or neutrophils per liter blood, preferably with at least 1 x 10⁹ lymphocytes or neutrophils per liter blood. This does not exclude transiently artifically reduced levels of lymphocytes or neutrophils within the course of the present invention to be excluded from the preferred embodiments of this invention.

Within the present invention certain critical baselines for lymphocytes or neutrophils numbers (which are within the physiological range) are contemplated, for example to define a low neutrophil number or a high lymphocyte number, or combinations of these values, such as the difference between neutrophil count and lymphocyte count (neutrophils minus lymphocytes; NML) or neutrophils or lymphocytes low high (NOL-LH).

Specifically the parameter NOL-LH (low neutrophils or high lymphocytes for a good prognosis) is a surprising result, because although it is in principle beneficial to have a high lymphocyte number, but even if lymphocytes are low, good prognosis is even possible when the neutrophil count is low. On the other hand, it is beneficial to have a low neutrophil number, however, even if neutrophil numbers are high, prognosis is still good, if the lymphocyte numbers are high. Only if the lymphocyte numbers are low and neutrophil numbers are high, a very bad prognosis is given. Since neutrophils represent usually around 70 % of total leukocytes, the subject matter of the present invention also relates to methods as disclosed herein, where instead of neutrophils total leukocytes are contemplated, optionally with a correction factor of (around) 70 %, e.g. instead of 4.0 to 6.0, especially 5.0 x 10⁹ neutrophils per liter blood, one could also use 6.5 to 8.5, especially 7.5 x 10⁹ leukocytes per liter blood. However, there is a likelihood that a certain amount of significance is lost, if one relies on the total leukocyte number instead of neutrophil counts.

The present invention allows a statistically significant prognosis of the progression of the tumor disease. The clinical benefit according to the present invention may be defined in various established ways in tumor medicine. For example, definition is proper if based on time-to-event measurements (against a control group):
- Improvement in overall survival (fully accepted by all oncologists and regulatory authorities)
- Improvement in relapse-free survival (when no clinically evident tumor mass is present; i.e. after surgery; = adjuvant setting)
- Improvement in progression-free survival (time until disease is progressing, based on defined measurable increase in tumor mass).

Benefit also is often also claimed in case of an improved response rate (based on defined shrinking of a tumor mass for a certain time period). However, clinical response in terms of tumor shrinking does not necessarily improve survival parameters. According to a preferred embodiment, the improved clinical benefit is an improved overall survival time.

As already stated above, the method according to the present invention is preferably performed only with individuals having a number of neutrophils or lymphocytes within "normal" ranges, i.e. usually above 1.0 x 10⁹ of each cell type per liter blood. Preferably the number of lymphocytes is from above 1.6 x 10⁹ to 5.0 x 10⁹ per liter blood for a good prognosis, especially from 1.6 x 10⁹ to 4.5 x 10⁹ per liter blood. According to a preferred embodiment, the number of neutrophils for a good prognosis is from 1.0 x 10⁹ to 5.0 x 10⁹ per liter blood, especially from 1.7 x 10⁹ to 5.0 x 10⁹ per liter blood.

According to another aspect, the present invention also relates to a method for identifying the prognosis for improved clinical benefit of an individual suffering from a tumor comprising
- providing a blood sample of said individual,
- (a1) determining the number of lymphocytes in the blood of said individual and/or
- (a2) determining the number of neutrophils in the blood of said individual and
- (b1) identifying the individual as having a good prognosis for improved clinical benefit, if the number of lymphocytes is above a lymphocyte baseline level of 1.4 to 1.8 x 10⁹ per liter blood, especially above 1.6 x 10⁹ per liter blood; or
- (b2) identifying the individual as having a good prognosis for improved clinical benefit, if the number of neutrophils is below or equal to a neutrophil baseline level of from 4.0 to 6.0 x 10⁹ per liter blood, especially below or equal to 5.0 x 10⁹ neutrophils per liter blood; or
- (b3) identifying the individual as having a bad prognosis for improved clinical benefit, if the number of lymphocytes is below or equal to a lymphocyte baseline level of 1.4 to 1.8 x 10⁹ per liter blood, especially below or equal to 1.6 x 10⁹ per liter blood and the number of neutrophils is above a neutrophil baseline level of from 4.0 to 6.0 x 10⁹ per liter blood, especially above 5.0 x 10⁹ neutrophils per liter blood.

The methods according to the present invention are generally applicable for all types of tumor patients, specifically for tumor patients undergoing tumor therapy. The present invention is therefore specifically suited for solid tumors, especially for tumors of the colorectal system. The present method is surprisingly suitable for tumors which are non-immunogenic tumors (i.e. tumors with low frequency of spontaneous remission; in contrast to immunogenic tumors, such as melanoma or renal cell carcinoma). Preferred tumors for the method according to the present invention are tumors which are non-immunogenic tumors (and therefore do not have a direct correlation to white blood cells), especially epithelial tumors. These preferred tumors have an extremely low likelihood for spontaneous remission mainly, because these tumors are more "hidden" to the immune system. Examples of preferred tumors according to the present invention are breast cancer, lung cancer, especially Non-Small-Cell-Lung-Cancer (NSCLC), cancer in the stomach, pancreas carcinoma, prostate cancer, ovarial carcinoma and colorectal cancer. The present method is preferably applied for patients with colorectal cancer.

Preferably, the prognosis is made of a tumor patient in stage III or IV of said tumor, classified according to the American Joint Committee on Cancer Manual for Staging Cancer, 6th Edition (2002), especially a tumor patient in stage IV.

The neutrophil or lymphocyte number may also be adjusted to a level for good prognosis, if the original level of the individual does not indicate good prognosis. Therefore, the cancer patient individual is an individual who had received a neutrophil reduction treatment, preferably selected from one or more of neutrophil reducing apheresis, radiation therapy or administration of neutrophil reducing agents, especially of chemotherapeutic drugs, monoclonal antibodies against neutrophil markers, immunomodulatory drugs, cytokines or mixtures thereof. With such suitable methods, it is possible to modulate neutrophil values in peripheral blood e.g. to or below or equal to 5.0 x 10⁹ per liter blood, respectively. Such a transient modulation of the WBC counts of cancer patients could e.g. be done immediately before start of cancer therapy, especially before cancer immunotherapy (against all common understanding of the problems caused by a damaged immune system for cancer vaccination) and may then lead to a survival benefit. As stated above, this could be achieved by several means:
- Apheresis techniques to transiently reduce neutrophil counts
- Radiation therapies to transiently reduce neutrophil counts
- Pre-treatment with agents that reduce the number of neutrophils, such as chemotherapeutic drugs, monoclonal antibodies against lymphocyte markers (e.g. OKT-3, anti- CD25 Mabs), immunomodulatory drugs, cytokines or mixtures thereof in an amount to effectively bring the neutrophil number to the desired level for good prognosis.

Alternatively, the cell number, especially the number of lymphocytes may be raised by suitable methods. It may therefore be preferred if the individual is an individual who had received a lymphocyte number enhancement treatment, preferably IL-2 administration or lymphocyte administration.

In a preferred embodiment according to the present invention, a method for identifying the prognosis for improved clinical benefit of an individual suffering from a tumor is provided which comprises
- providing a blood sample of said individual,
- (a1) determining the number of lymphocytes in the blood of said individual, and
- (a2) determining the number of neutrophils in the blood of said individual and
- (b1) identifying the individual as having a good prognosis for improved clinical benefit, if the number of lymphocytes is above a lymphocyte baseline level of 1.4 to 1.8 x 10⁹ per liter blood, especially above 1.6 x 10⁹ per liter blood (even if the number of neutrophils is above 4.0 to 6.0 x 10⁹ per liter blood, especially above 5.0 x 10⁹ neutrophils per liter blood); or
- (b2) identifying the individual as having a good prognosis for improved clinical benefit, if the number of neutrophils is below or equal to a neutrophil baseline level of from 4.0 to 6.0 x 10⁹ per liter blood, especially below or equal to 5.0 x 10⁹ neutrophils per liter blood (even if the number of lymphocytes is below or equal to 1.4 to 1.8 x 10⁹ per liter blood, especially below or equal to 1.6 x 10⁹ per liter blood); or
- (b3) identifying the individual as having a bad prognosis for improved clinical benefit, if the number of lymphocytes is below or equal to a lymphocyte baseline level of 1.4 to 1.8 x 10⁹ per liter blood, especially below or equal to 1.6 x 10⁹ per liter blood and the number of neutrophils is above a neutrophil baseline level of from 4.0 to 6.0 x 10⁹ per liter blood, especially above 5.0 x 10⁹ neutrophils per liter blood.

A preferred parameter within the scope of the present invention is a composite parameter based on the difference of the neutrophil number and the lymphocyte number. This parameter has proven to have a very good prognosis efficacy. Accordingly, it is preferred that the difference of the neutrophil number and the lymphocyte number (neutrophils minus lymphocytes; NML) is determined and
- (b4) identifying the individual as having a good prognosis for improved clinical benefit, if the NML is below or equal to an NML baseline level of from 2,4 to 4,4 x 10⁹ cells per liter blood, especially below or equal to 3,4 x 10⁹ cells per liter blood; or
- (b5) identifying the individual as having a bad prognosis for improved clinical benefit, if the NML is above an NML baseline level of from 2,4 to 4,4 x 10⁹ cells per liter blood, especially above 3,4 x 10⁹cells per liter blood.

The present method is may be suitable for designing and evaluating clinical trials for proving efficacy of a given tumor treatment by grouping of individuals in specific groups selected and determined according to the parameters at baseline according to the present invention. This results in an efficient grouping which is not based on an unknown and therefore unconsidered prognosis factor of a group which would otherwise make a proper evaluation of the cancer treatment difficult. This grouping can be done also retrospectively, because lymphocyte numbers and neutrophil numbers are measured on a routine basis in all clinical trials involving tumor patients. Results of controlled clinical trials may be corrected for possible imbalances regarding the here described prognostic factors using statistical methods such as Cox' regression analysis. According to a specific aspect, the present invention therefore also relates to a method for conducting clinical trials for a tumor treatment comprising the grouping of individuals into a group which has a lymphocyte number above a lymphocyte baseline level and a group which has a lymphocyte number below or equal to a lymphocyte baseline level, said lymphocyte baseline level being a value from 1.4 to 1.8 x 10⁹ lymphocytes per liter blood, especially 1.6 x 10⁹ per liter blood.

According to another aspect, the present invention relates to a method for identifying an individual suffering from a tumor as having a good prognosis for improved overall survival time comprising
- providing a blood sample of said individual,
- determining the number of neutrophils and the number of lymphocytes in the blood of said individual, and
- identifying the individual as having a good prognosis for improved overall survival time, if the difference of the number of neutrophils and the number of lymphocytes is below or equal to a neutrophil-minus-lymphocyte baseline level of from 3.0 to 4.0 x 10⁹ per liter blood, especially below or equal to 3.4 x 10⁹ per liter blood.

As already mentioned above, this method is preferred in patients having colorectal cancer. This method is specifically suited for patients in later stages of the disease, for example tumor patients in stage III or IV of said tumor, classified according to the American Joint Committee on Cancer Manual for Staging Cancer, 6th Edition (2002), especially a tumor patient in stage IV.

The present invention is further described in the following examples and figures, yet without being restricted thereto.
Fig. 1 shows the influence of baseline neutrophil counts on overall survival for all placebo patients;
Fig.2 shows the influence of baseline neutrophil counts on overall survival for all stage IV placebo patients;
Fig.3 shows the influence of baseline neutrophil counts on overall survival for all stage IV placebo patients with colorectal carcinoma;
Fig.4 shows the influence of baseline lymphocyte counts on overall survival for all placebo patients;
Fig.5 shows the influence of baseline lymphocyte counts on overall survival for all stage IV placebo patients;
Fig.6 shows the influence of baseline lymphocyte counts on overall survival for all stage IV placebo patients with colorectal carcinoma;
Fig.7 shows the influence of baseline neutrophil minus lymphocyte counts on overall survival for placebo patients;
Fig.8 shows the influence of baseline neutrophil minus lymphocyte counts on overall survival for all stage IV placebo patients;
Fig.9 shows the influence of baseline neutrophil minus lymphocyte counts on overall survival for all stage IV placebo patients with colorectal carcinoma;
Fig.10 shows shows the influence of baseline neutrophil or lymphocyte counts on overall survival for placebo patients;
Fig.11 shows the influence of baseline neutrophil or lymphocyte counts on overall survival for all stage IV placebo patients;
Fig.12 shows the influence of baseline neutrophil or lymphocyte counts on overall survival for all stage IV placebo patients with colorectal carcinoma;
Fig. 13 shows the influence of neutrophil counts at relapse on overall survival for all relapsed patients with NSCLC;
Fig. 14 shows the influence of lymphocyte counts at relapse on overall survival for all relapsed patients with NSCLC;
Fig. 15 shows the influence of neutrophil minus lymphocyte counts at relapse on overall survival for all relapsed patients with NSCLC;
Fig. 16 shows the influence of neutrophil counts at baseline on relapse-free survival for all patients with NSCLC stages Ib, II and IIIa;
Fig. 17 shows the influence of neutrophil counts at baseline on overall survival for all patients with NSCLC stages Ib, II and IIIa;
Fig. 18 shows the influence of neutrophil minus lymphocyte counts at baseline on relapse-free survival for all patients with NSCLC stages Ib, II and IIIa; and
Fig. 19 shows the influence of neutrophil minus lymphocyte counts at baseline on overall survival for all patients with NSCLC stages Ib, II and IIIa.

### Examples:

The present investigations are based on a data set accumulated in the context of a finished and thus opened double-blind placebo controlled Phase IIb trial with the cancer vaccine IGN101 (Himmler et al., Proceedings ASCO 2005, Abstract # 2555, "A randomized placebo-controlled phase II study with the cancer vaccine candidate IGN101 in patients with epithelial cancers") in patients with epithelial cancers. For the present analyses, only data of patients randomised to placebo were used in order to rule out any possible influence of the tested vaccine IGN101 on the further disease course. Therefore, the results are assumed to be representative. As for the induction of an immune response white blood cells are crucially important, the investigations described in this example utilise standard white blood cell counts, in particular lymphocyte and neutrophil counts.

### Methods:

For the phase IIb, placebo-controlled trial to evaluate the efficacy, safety, tolerability and immunogenicity of multiple doses of IGN101, the present values for the leukocyte, neutrophil and lymphocyte counts were analysed. In general, analyses are based on the Intention To Treat (ITT) population. The actually used number of patients is always mentioned in the respective section. These numbers may differ to a minor extent from the ITT population as defined in the official study report, since (very few) patients with missing baseline parameters or death date information had to be omitted.

For the calculations and plotting of figures the following software programmes were used:
- Tables: MS Excel
- Kaplan Meier survival curves: GraphPad Prism 4
- Cox regression analysis: Internet:
   http://members.aol.com/johnp71/prophaz2.html

### Results:

Analysis of parameters with possible prognostic value for OS

### 1. All placebo patients: Neutrophil counts at baseline

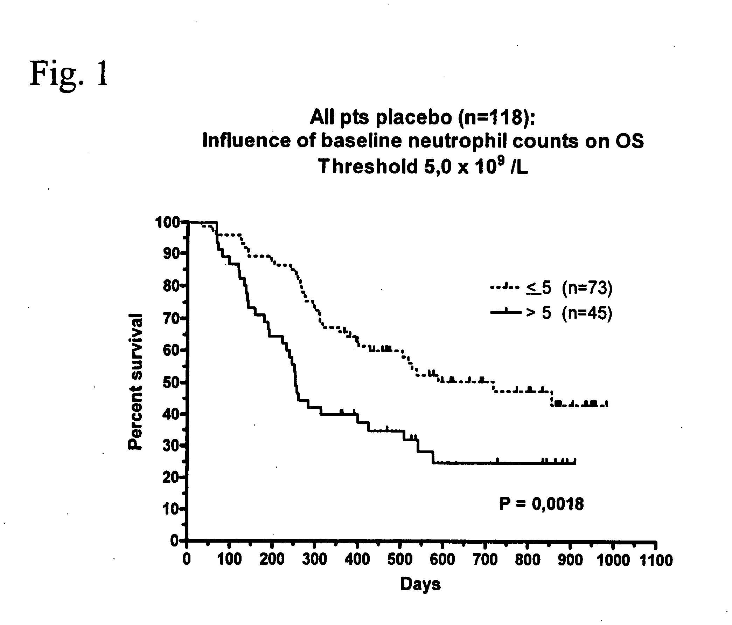

The majority of leukocytes in peripheral blood are neutrophils. Patients were separated into two groups with a threshold of 5,0 x 10⁹ / L neutrophils measured at baseline. The respective Kaplan-Meier results for all placebo patients are shown in Fig. 1.

The observed difference of the survival curves is highly statistically significant (P = 0,0018 log-rank; median survival 253 vs 717 days). Stage III patients (who in general have a longer OS than stage IV patients) predominantly are in the group with lower neutrophil numbers. The distribution of patients in the two analysed groups is as follows:

| | | < 5 | | > 5 | |
|---|---|---|---|---|---|
| Stage III: | | 43 | 36.4 % | 11 | 9.3 % |
| | colon: | 18 | | 5 | |
| | rectum: | 12 | | 1 | |
| | NSCLC: | 5 | | 5 | |
| | gastric: | 6 | | 0 | |
| | other: | 2 | | 0 | |
| | | | | | |
| Stage IV: | | 30 | 25.4 % | 34 | 28.8 % |
| colon: | | 17 | | 8 | |
| | rectum: | 8 | | 13 | |
| | NSCLC: | 1 | | 8 | |
| | gastric: | 3 | | 4 | |
| | other: | 1 | | 1 | |

In Cox' regression analysis with KPS, neutrophil count at baseline and stage (III or IV) as parameters, the neutrophil count is a statistically significant independent predictor for OS. Lower neutrophil counts at baseline are associated with improved survival:

| Var | Coeff. | StdErr | p | Lo95% | Hi95% |
|---|---|---|---|---|---|
| Neutrophils | 0.1133 | 0.0514 | 0.0276 | 0.0130 | 0.2135 |
| KPS | -0.0887 | 0.0232 | 0.0001 | -0.1340 | -0.0433 |
| Stage | 1.3042 | 0.3056 | 0.0000 | 0.7083 | 1.9002 |

### 2. Placebo patients stage IV: Neutrophil counts at baseline

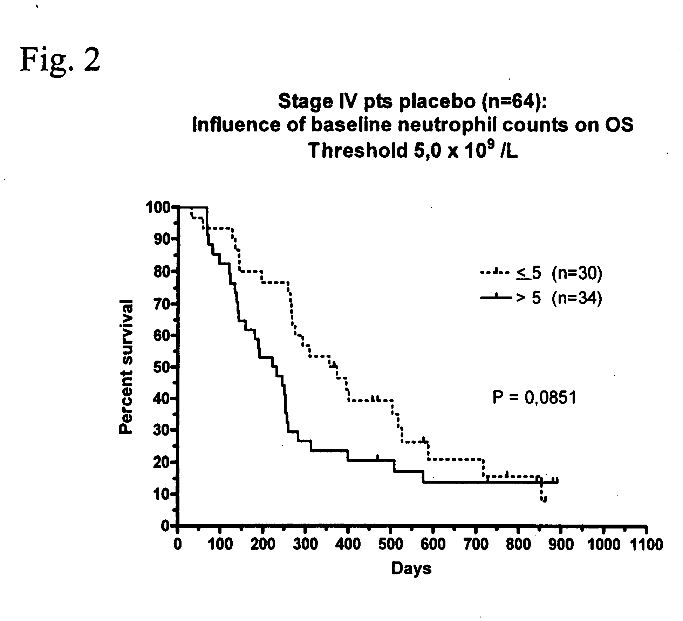

Next, the influence of the neutrophil count at baseline on OS of the stage IV patients only was evaluated, using the same principles as described in 1. The Kaplan-Meier results are shown in Figure 2.

The difference of the survival curves is close to statistical significance (P = 0.0851 log-rank; median survival 227 vs 365 days). In Cox' regression analysis, however, with KPS and neutrophil count at baseline as parameters in order to correct for the influence of KPS on OS, the neutrophil count turns out to be a highly statistically independent predictor for OS. Lower neutrophil counts at baseline are associated with improved survival:

| Var | Coeff. | StdErr | p | Lo95% | Hi95% |
|---|---|---|---|---|---|
| Neutrophils | 0.1599 | 0.0586 | 0.0064 | 0.0456 | 0.2742 |
| KPS | -0.0783 | 0.0265 | 0.0032 | -0.1300 | -0.0266 |

### 3. Placebo patients with colo-rectal cancer stage IV: Neutrophil counts at baseline

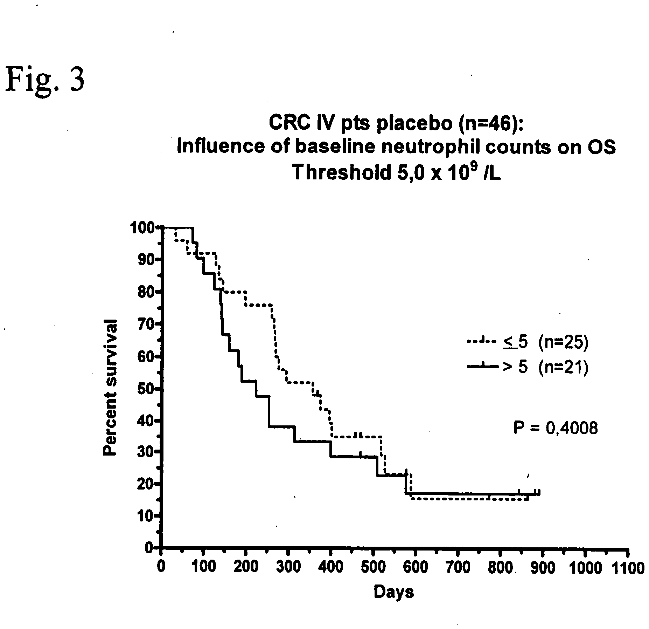

The influence of the neutrophil count at baseline on OS of the colo-rectal cancer patients stage IV patients was evaluated as well, using the same principles as described in 1. The respective Kaplan-Meier results are shown in Figure 3.

According to this Kaplan-Meier analysis, the observed minor differences of the survival curves are not significant. However, when the data are corrected for a possible influence of KPS on OS by Cox' regression analysis with neutrophil counts at baseline and KPS as descriptors, the neutrophil count again appears as a significant independent predictor of OS. Lower neutrophil numbers relate to better OS:

| Var | Coeff. | StdErr | p | Lo95% | Hi95% |
|---|---|---|---|---|---|
| Neutrophils | 0.1961 | 0.0799 | 0.0141 | 0.0403 | 0.3520 |
| KPS | -0.0644 | 0.0298 | 0.0307 | -0.1225 | -0.0063 |

### 4. All placebo patients: Lymphocyte counts at baseline

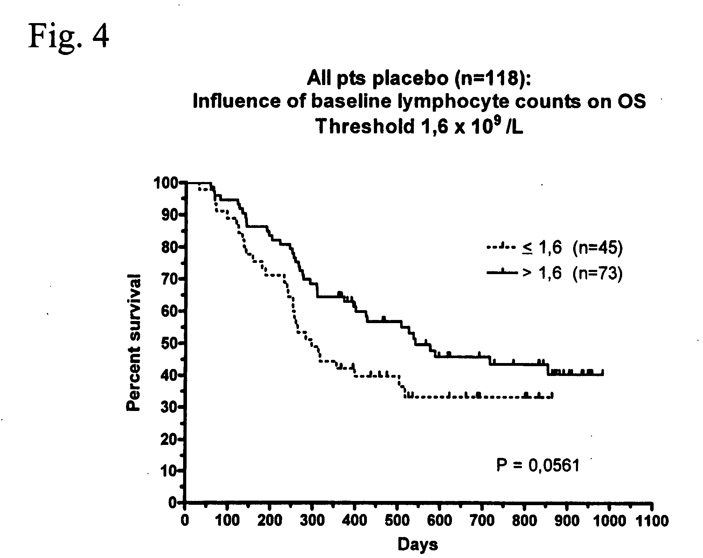

The leukocytes of the peripheral blood in general consist of neutrophils (usually the largest fraction), monocytes and lymphocytes. Since the lymphocytes (in particular B cells and T cells) are especially important for the establishment of an immune response and to maintain immunity, a set of analyses as described in the previous section was also done with the lymphocyte counts at baseline. For this, patients were separated into two groups with a threshold of 1.6 x 10⁹ / L lymphocytes measured at baseline. The respective Kaplan-Meier results for all placebo patients are shown in Fig. 4.

The difference of the survival curves is almost statistically significant. In contrast to the results obtained with neutrophils and leukocytes, where lower cell counts at baseline were associated with longer OS, according to this analysis patients apparently have a longer OS when presenting with higher lymphocyte counts at baseline. The distribution of patients in the two analysed groups is as follows:

| | | < 1.6 | | | > 1.6 |
|---|---|---|---|---|---|
| Stage III: | | 18 | 15.3 % | 36 | 30.5 % |
| | colon: | 7 | | 16 | |
| | rectum: | 6 | | 7 | |
| | NSCLC: | 3 | | 7 | |
| | gastric: | 2 | | 4 | |
| | other: | 0 | | 2 | |
| | | | | | |
| Stage IV: | | 27 | 22.9 % | 37 | 31.4 % |
| colon: | | 5 | | 20 | |
| | rectum: | 12 | | 9 | |
| | NSCLC: | 6 | | 3 | |
| | gastric: | 4 | | 3 | |
| | other: | 0 | | 2 | |

However, in Cox' regression analysis with KPS, lymphocyte count at baseline and stage (III or IV) as parameters, the lymphocyte count does not appear as significant independent predictor for OS, only KPS and stage are independent predictors. Likely, a lower lymphocyte count is associated with a lower KPS:

| Var | Coeff. | StdErr | p | Lo95% | Hi95% |
|---|---|---|---|---|---|
| Lymphocytes | -0.1267 | 0.1662 | 0.4457 | -0.4508 | 0.1973 |
| KPS | -0.0922 | 0.0233 | 0.0001 | -0.1377 | -0.0468 |
| Stage | 1.3371 | 0.3061 | 0.0000 | 0.7402 | 1.9340 |

### 5. Placebo patients stage IV: Lymphocyte counts at baseline

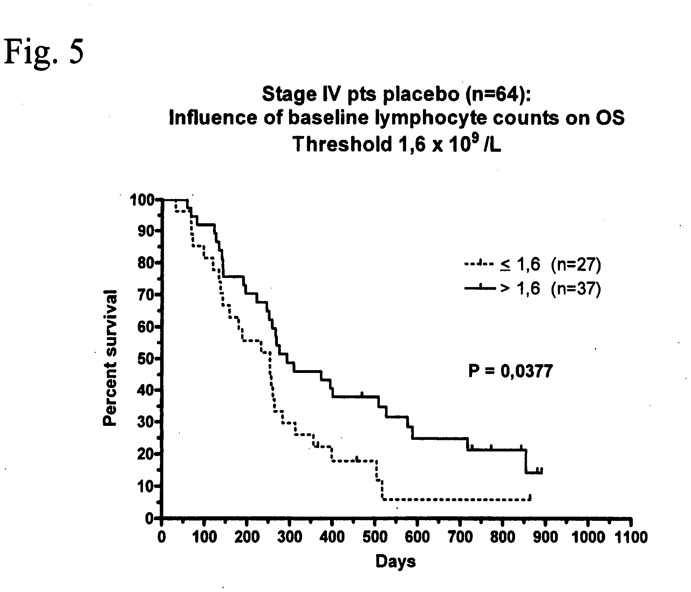

A similar analysis was done in the more homogeneous group of all stage IV patients of the placebo group. The Kaplan-Meier results are shown in Fig. 5.

The observed differences of the survival curves are significant. However, In Cox' regression analysis with KPS and lymphocyte count at baseline as parameters, the lymphocyte count does not appear as significant independent predictor for OS, a trend is still seen. Obviously, KPS and lymphocyte count have a correlation:

| Var | Coeff. | StdErr | p | Lo95% | Hi95% |
|---|---|---|---|---|---|
| 1 | -0.0809 | 0.0264 | 0.0022 | -0.1324 | -0.0294 |
| 2 | -0.2702 | 0.1967 | 0.1695 | -0.6537 | 0.1134 |

### 6. Placebo patients with colo-rectal cancer stage IV: Lymphocyte counts at baseline

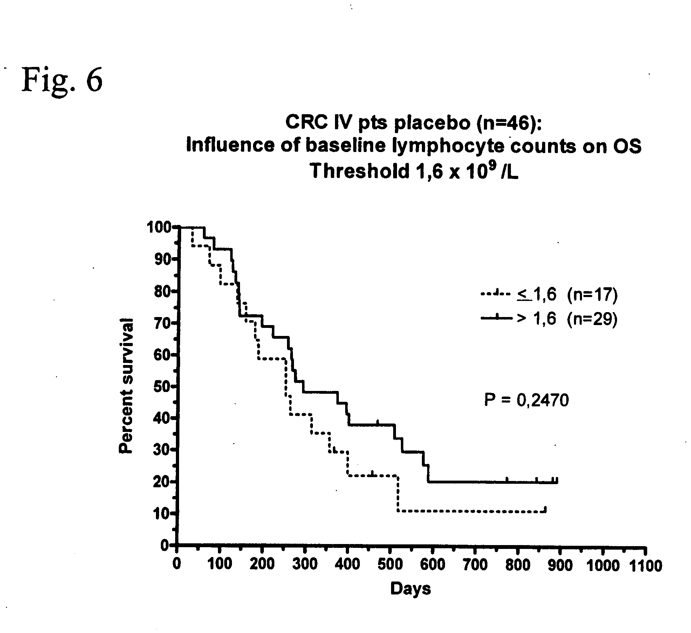

The Kaplan-Meier results of the analysis of the CRC IV patients as most homogeneous group are shown in Fig. 6.

The observed difference of the survival curves is not significant. Also in Cox' regression analysis with KPS and lymphocyte count at baseline as parameters, the lymphocyte count does not appear as significant independent predictor for OS, a trend, however, is still seen.

| Var | Coeff. | StdErr | p | Lo95% | Hi95% |
|---|---|---|---|---|---|
| KPS | -0.0656 | 0.0311 | 0.0350 | -0.1263 | -0.0049 |
| lympho | -0.3323 | 0.2358 | 0.1589 | -0.7922 | 0.1276 |

### 7. All placebo patients: Baseline neutrophil minus lymphocyte counts

According to the results described so far, both the neutrophil count and the lymphocyte count have a certain prognostic value, lower neutrophil counts and higher lymphocyte counts relate to improved OS. An also observed prognostic value of the summary leukocyte count (lower leukocyte counts relate to improved OS) therefore is mostly determined by the neutrophil count.

In order to use the lymphocyte and neutrophil results optimally in one parameter, the difference of the neutrophil count and the lymphocyte count as new parameter is introduced (abbreviation NML for Neutrophils Minus Lymphocytes). Although NML probably has no direct biological correlate, it should strengthen the prognostic potential, as a low values for neutrophils and high values for lymphocytes are required for better OS. The threshold of 3.4 x 10⁹ / L used for NML was derived from the thresholds used for the single analyses [(5.0 - 1.6) x 10⁹ / L].

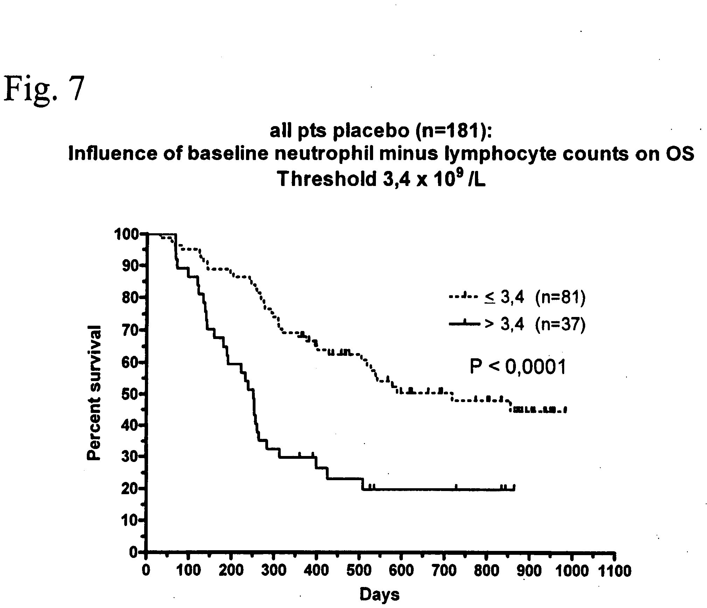

The Kaplan-Meier results for all placebo patients, using baseline NML as prognostic parameter, are shown in Figure 7.

As expected, this parameter led to a pronounced differences in OS. Similarly, high significance was also found in Cox' regression. Lower values of NML are strongly correlated with improved OS.

| Var | Coeff. | StdErr | p | Lo95% | Hi95% |
|---|---|---|---|---|---|
| KPS | -0.0861 | 0.0234 | 0.0002 | -0.1317 | -0.0404 |
| NML | 0.1245 | 0.0516 | 0.0158 | 0.0239 | 0.2250 |
| stage | 1.2894 | 0.3059 | 0.0000 | 0.6930 | 1.8859 |

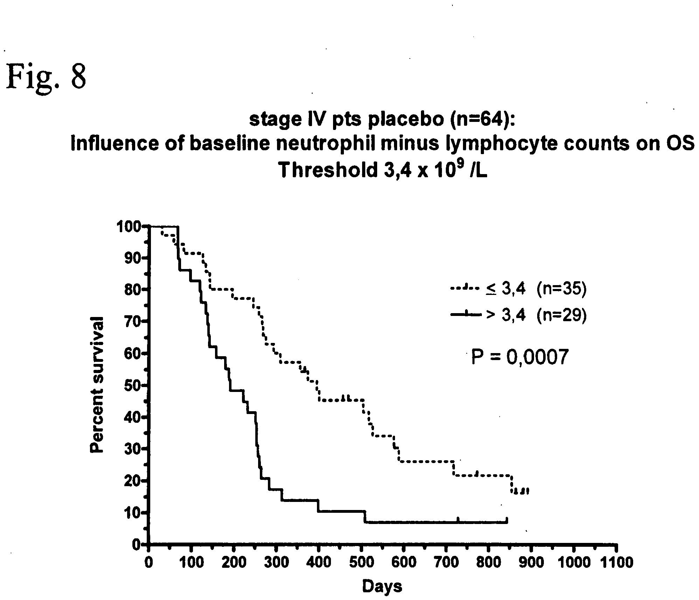

### 8. Placebo patients stage IV: NML counts at baseline A similar analysis as described in 7. was also performed for the stage IV patients of the placebo group. The Kaplan-Meier results are shown in Figure 8.

The results are highly significant. Similarly, in Cox' regression analysis with KPS and NML as predictors, NML retained as significant independent prognostic parameter. Lower values are associated with improved survival:

| Var | Coeff. | StdErr | p | Lo95% | Hi95% |
|---|---|---|---|---|---|
| KPS | -0.0756 | 0.0268 | 0.0048 | -0.1278 | -0.0233 |
| NML | 0.1715 | 0.0568 | 0.0025 | 0.0608 | 0.2823 |

### 9. Placebo patients with colo-rectal cancer stage IV: NML counts at baseline

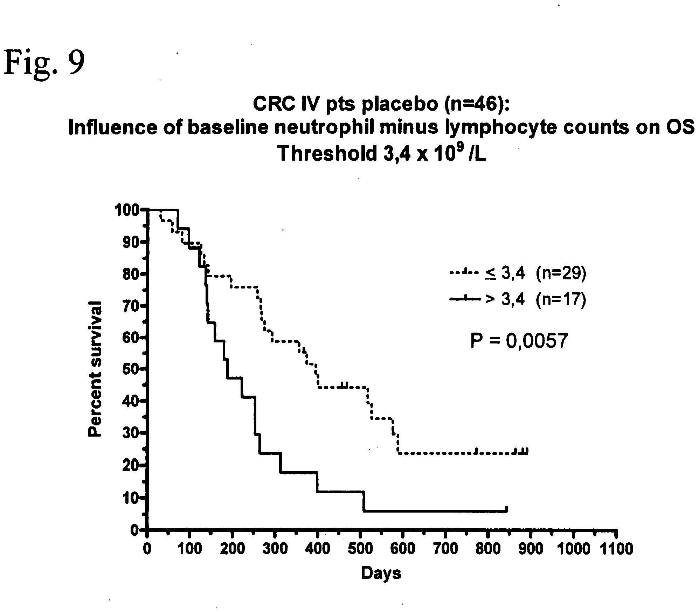

Analysis of the CRC IV patients of the placebo group regarding the prognostic value of NML led to very similar results as described above. The Kaplan-Meier survival curves are shown in Fig. 9.

The results again are highly significant. In the group with lower NML, the median survival time is more than doubled (395 d versus 188 d, hazard ratio 0.4162). Also the 1-year survival is more than doubled: 55.2% versus 17.6%. The slope of the two survival curves is different (as seen also in Figures 7 and 8), indicating a higher proportion of long-term survivors in the group with lower NML.

In Cox' regression analysis with KPS and NML as predictors, also for CRC IV patients NML retained as strongly significant independent prognostic parameter. Lower values are associated with improved survival:

| Var | Coeff. | StdErr | p | Lo95% | Hi95% |
|---|---|---|---|---|---|
| KPS | -0.0625 | 0.0302 | 0.0386 | -0.1214 | -0.0036 |
| NML | 0.2048 | 0.0762 | 0.0072 | 0.0563 | 0.3533 |

### 10. All placebo patients: Combination of low neutrophil or high lymphocyte counts at baseline

Another way of combining the prognostic values of low neutrophil and high lymphocyte counts at baseline is a combination as follows: Placebo patients were grouped according to a baseline lymphocyte count of > 1.6 x 10⁹ / L or a baseline neutrophil count of ≤ 5.0 x 10⁹ / L. In consequence, the remaining patients have a baseline lymphocyte count ≤ 1.6 x 10⁹ / L and a baseline neutrophil count > 5.0 x 10⁹ / L. This novel composite parameter is named NOL-LH (Neutrophils Or Lymphocytes-Low High).

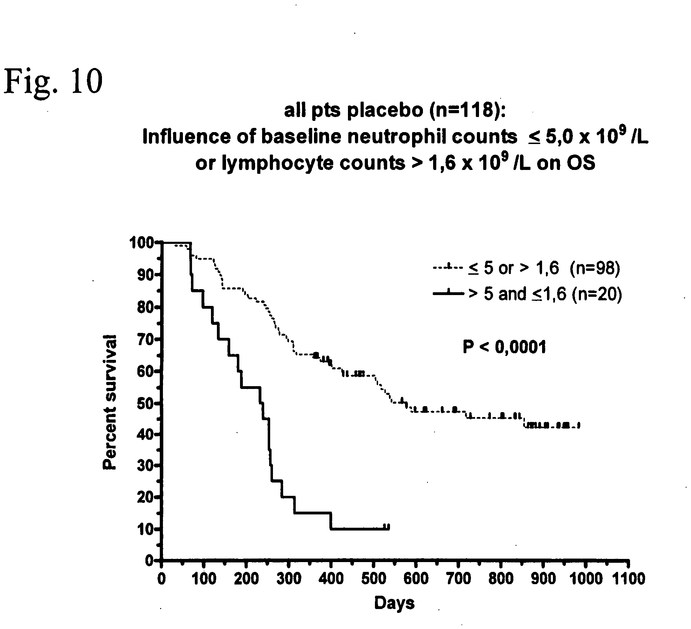

The respective Kaplan Meier result based on NOL-LH is shown in Fig. 10.

The difference of the survival curves is highly significant. Remarkably, more than 80% of the patients are in the group with improved survival. The use of NOL-LH seemingly leads to a precise selection of patients with substantially worse prognosis. The distribution of patients is as follows:

| | | ≤ 5 or > 1.6 | | > 5 and ≤ 1.6 | |
|---|---|---|---|---|---|
| Stage III: | | 51 | 43.2 % | 3 | 2.9 % |
| | colon: | 22 | | 1 | |
| | rectum: | 13 | | 0 | |
| | NSCLC: | 8 | | 2 | |
| | gastric: | 6 | | 0 | |
| | other: | 2 | | 0 | |
| | | | | | |
| Stage IV: | | 46 | 39.0 % | 18 | 15.3 % |
| colon: | | 23 | | 2 | |
| | rectum: | 13 | | 8 | |
| | NSCLC: | 4 | | 5 | |
| | gastric: | 4 | | 3 | |
| | other: | 2 | | 0 | |

In stage III, 51 of 54 patients (94.4%) belong to the group with good prognosis. This is in agreement with the generally better prognosis of stage III patients and indicates the power of the composite parameter NOL-LH to discriminate between patients with good and bad prognosis, and even "recognise" tumor stages with favorable prognosis, only based on the information of a certain pattern of the white blood cell counts regarding neutrophils and lymphocytes.

### 11. Placebo patients stage IV: Combination of low neutrophil or high lymphocyte counts at baseline

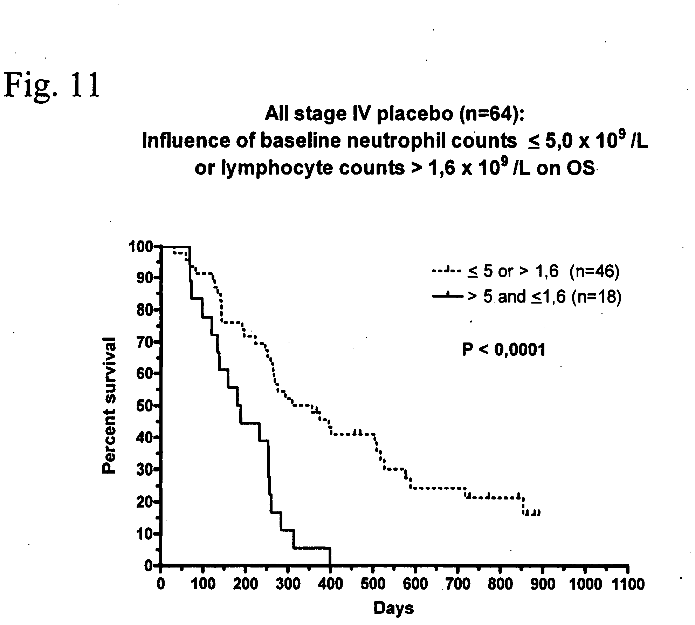

A similar analysis as described in 10 was also done for the placebo stage IV patients only. The Kaplan-Meier results are shown in Figure 11.

The results show a highly significant survival difference and are very similar to those described in 10. More than 70% of patients are in the group with better prognosis, again indicating the power of NOL-LH to select patients with bad prognosis.

### 12. Placebo patients with colo-rectal cancer stage IV: Combination of low neutrophil or high lymphocyte counts at baseline

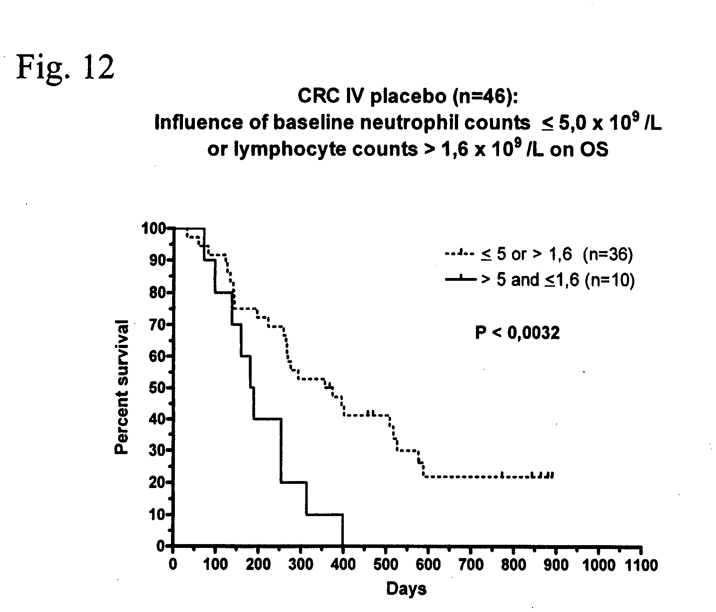

Finally, this kind of analysis was applied for the placebo CRC IV patients. The Kaplan Meier results are shown in Figure 12: The use of the composite parameter NOL-LH for selection of CRC IV patients again leads to a highly significant difference of the survival curves. In the group with improved prognosis the median survival is doubled (365 days vs 184 days), and the 1-year survival is 5-times higher (50% vs 10%). More than 75% of all CRCIV patients are in the group with improved survival, again indicating the power of NOL-LH to precisely identify CRC IV patients with bad prognosis.

### 13. Summary of results regarding prognostic parameters The following table summarises the P values (Kaplan-Meier curves; log-rank) obtained with leukocyte-, neutrophil-, lymphocyte-, NML-, and NOL-LH counts at baseline regarding their ability to define patient groups with different overall survival.

| | | P values | | |
|---|---|---|---|---|
| | | all placebo pts | placebo stage IV | placebo CRC IV |
| | threshold (x10E9/L) | (n=118) | (n=64) | (n=46) |
| leukocytes | 7.5 | 0.0311 | 0.1297 | 0.5694 |
| neutrophils | 5 | 0.0018 | 0.0851 | 0.4008 |
| lymphocytes | 1.6 | 0.0561 | 0.0377 | 0.247 |
| **NML** | **3.4** | **<0.0001** | **0.0007** | **0.0057** |
| **NOL-LH** | **5 or 1.6** | **<0.0001** | **<0.0001** | **0.0032** |

NOL-LH is the best parameter to prospectively define patient groups with substantially different prognosis for overall survival, with high statistical significance in all tested patient groups, closely followed by NML. Both NOL-LH and NML are composite parameters regarding neutrophil and lymphocyte counts, reflecting the results obtained with each of the single parameters (lower neutrophil counts and higher lymphocyte counts relate to improved OS).

### 14. Placebo-controlled clinical trial in patients with NSCLC, stages Ib, II and IIIa

Furthermore, a placebo-controlled double blind clinical trial with the cancer vaccine IGN101 in 762 patients with Non-Small-Cell-Lung-Cancer (NSCLC) stages Ib, II and IIIa was used to analyze above described prognostic parameters. As the study was still blinded, all patients (regardless of placebo or vaccine treatment) were used. At the time of analysis the study had included all patients and was ongoing for approx. 5 years. As in this study patients are included directly after R0-resection of their lung cancer, both relapse-free and overall survival (RFS and OS) is evaluated. In consequence, the prognostic parameters were both assessed regarding their prognostic value for RFS and OS. Neutrophil counts and lymphocyte counts were measured at entry for all patients and at the time of relapse for relapsed patients.

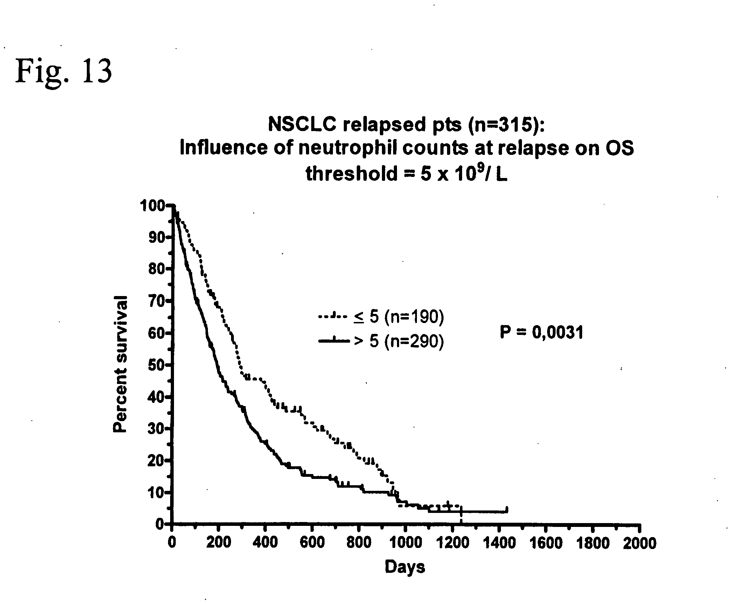

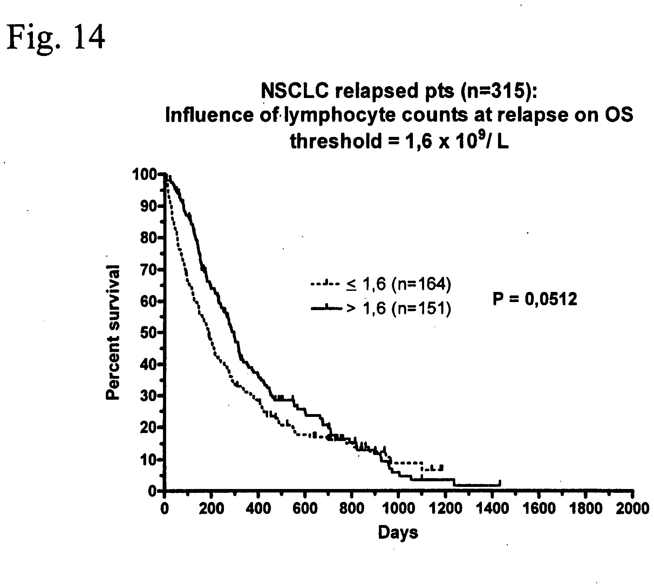

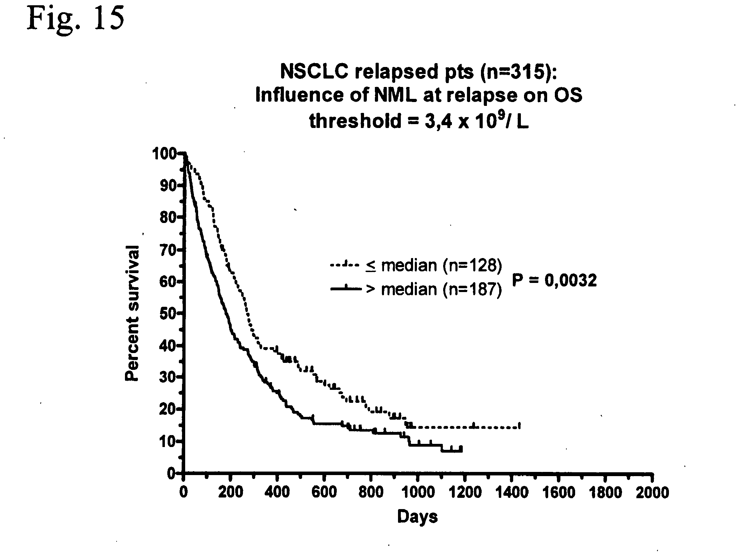

As shown in Figures 13-15, neutrophil counts, lymphocyte counts and NML at relapse have a similar prognostic value for overall survival as described above: Higher neutrophil counts and lower lymphocyte counts are indicators of bad prognosis. Higher NML also is a sign of bad prognosis.

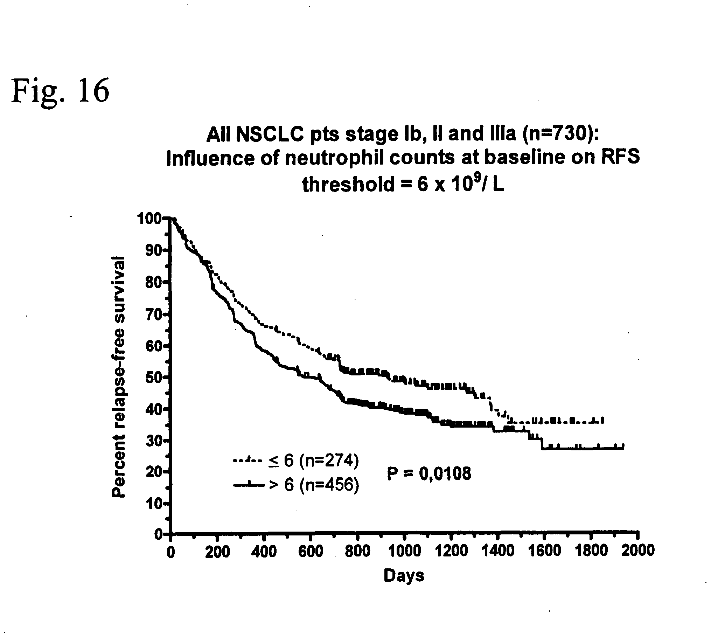

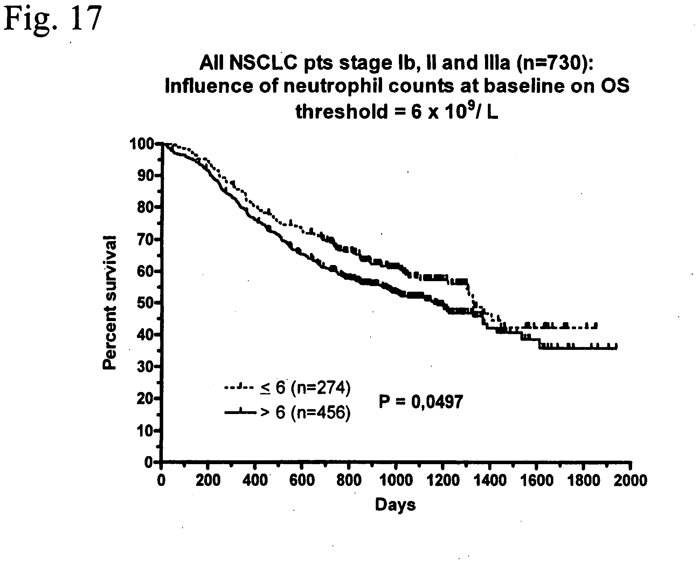

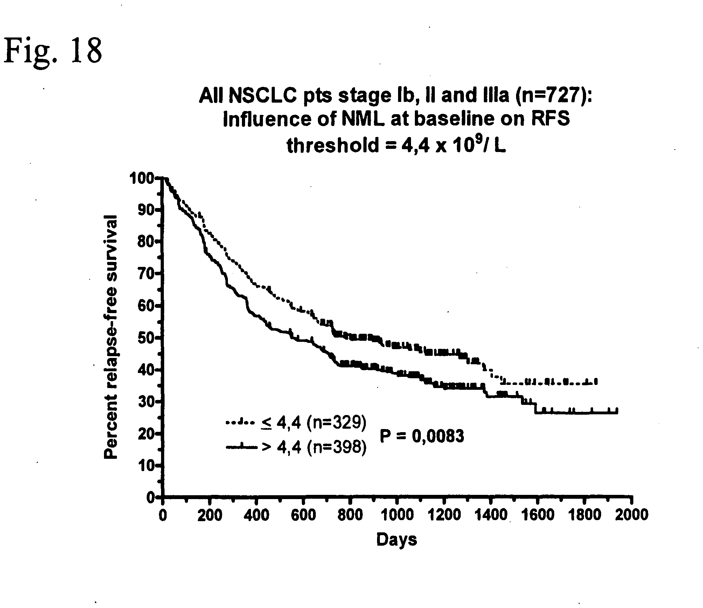

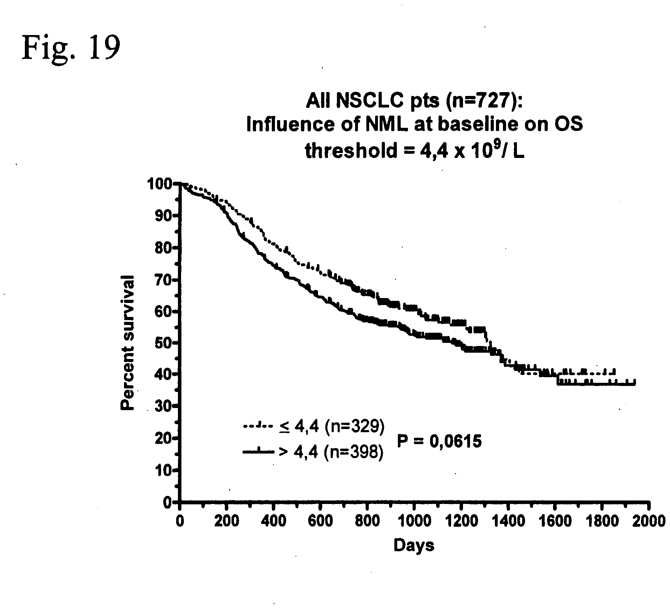

Strikingly, neutrophil counts and NML measured at entry of the NSCLC patients, i.e. directly after surgery, in the early stages Ib, II and IIa, also have a substantial prognostic value, both for RFS and OS. This is shown in Figures 16-19. Higher neutrophil counts and higher NML are signs of bad prognosis regarding RFS and OS. It is especially surprising that white blood cell counts directly after surgery of early stage NSCLC patients already have prognostic value for overall survival, as death of these patients due to lung cancer usually occurs several years after surgery (median in this study approx. 3 years after surgery) and usually after a relapse.

## Claims

1. A method for identifying the prognosis for improved clinical benefit of an individual suffering from an epithelial tumor comprising
- (a1) determining the number of lymphocytes in a blood sample of said individual in vitro, and
- (a2) determining the number of neutrophils in a blood sample of said individual in vitro, and
- (b) identifying the individual as having a bad prognosis for improved clinical benefit, if the number of lymphocytes is below or equal to a lymphocyte baseline level of 1.4 to 1.8 x 10⁹ per liter blood, especially below or equal to 1.6 x 10⁹ per liter blood and the number of neutrophils is above a neutrophil baseline level of from 4.0 to 6.0 x 10⁹ per liter blood, especially above 5.0 x 10⁹ neutrophils per liter blood.

2. A method according to claim 1, **characterised in that** the improved clinical benefit is an improved overall survival time.

3. A method according to claim 1 or 2, **characterised in that** said epithelial tumor is breast cancer, lung cancer, especially Non-Small-Cell-Lung-Cancer (NSCLC), cancer in the stomach, pancreas carcinoma, prostate cancer, ovarial carcinoma or colorectal cancer, especially colorectal cancer.

4. A method according to any one of claims 1 to 3, **characterised in that** said individual is a tumor patient in stage III or IV of said tumor, classified according to the American Joint Committee on Cancer Manual for Staging Cancer, 6th Edition (2002), especially a tumor patient in stage IV.

5. A method according to any one of claims 1 to 4, **characterised in that** said individual is an individual who had received a neutrophil reduction treatment, preferably selected from one or more of neutrophil reducing apheresis, radiation therapy or administration of neutrophil reducing agents, especially of chemotherapeutic drugs, monoclonal antibodies against neutrophil markers, immunomodulatory drugs, cytokines or mixtures thereof.

6. A method according to any one of claims 1 to 5, **characterised in that** said individual is an individual who had received a lymphocyte number enhancement treatment, preferably IL-2 administration or lymphocyte administration.

7. A method according to any one of claims 1 to 6, **characterised in that** the difference of the neutrophil number and the lymphocyte number (neutrophils minus lymphocytes; NML) is determined and
- (b4) identifying the individual as having a good prognosis for improved clinical benefit, if the NML is below or equal to an NML baseline level of from 2.4 to 4.4 x 10⁹ cells per liter blood, especially below or equal to 3.4 x 10⁹ cells per liter blood; or
- (b5) identifying the individual as having a bad prognosis for improved clinical benefit, if the NML is above an NML baseline level of from 2.4 to 4.4 x 10⁹ cells per liter blood, especially above 3.4 x 10⁹ cells per liter blood.

## Patentansprüche

1. Verfahren zur Feststellung der Prognose eines erhöhten klinischen Nutzens für ein Individuum, das an einem epithelialen Tumor erkrankt ist, welches Verfahren umfasst:
- (a1) Bestimmen der Anzahl der Lymphozyten in einer Blutprobe des Individuums in vitro, und
- (a2) Bestimmen der Anzahl der Neutrophilen in einer Blutprobe des Individuums in vitro, und
- (b) Identifizieren als ein Individuum mit schlechter Prognose für einen erhöhten klinischen Nutzen, wenn die Anzahl der Lymphozyten niedriger oder gleich einem Lymphozyten-Grundlinien-Level von 1,4 bis 1,8 x 10⁹ pro Liter Blut, insbesondere niedriger oder gleich 1,6 x 10⁹ pro Liter Blut ist und die Anzahl der Neutrophilen höher als ein Neutrophilen-Grundlinien-Level von 4,0 bis 6,0 x 10⁹ pro Liter Blut, insbesondere höher als 5,0 x 10⁹ Neutrophile pro Liter Blut ist.

2. Verfahren nach Anspruch 1, **dadurch gekennzeichnet, dass** der erhöhte klinische Nutzen eine verbesserte Gesamtüberlebenszeit ist.

3. Verfahren nach Anspruch 1 oder 2, **dadurch gekennzeichnet, dass** der epitheliale Tumor Brustkrebs, Lungenkrebs, insbesondere nicht-kleinzelliger Lungenkrebs (NSCLC), Magenkrebs, Pankreaskarzinom, Prostatakrebs, Ovarialkarzinom oder Kolorektalkrebs, insbesondere Kolorektalkrebs ist.

4. Verfahren nach einem der Ansprüche 1 bis 3, **dadurch gekennzeichnet, dass** das Individuum ein Tumorpatient in Stadium III oder IV des Tumors ist, klassifiziert gemäß dem American Joint Committee on Cancer Manual for Staging Cancer, 6th Edition (2002), insbesondere ein Tumorpatient in Stadium IV.

5. Verfahren nach einem der Ansprüche 1 bis 4, **dadurch gekennzeichnet, dass** das Individuum ein Individuum ist, das eine Behandlung zur Reduktion der Neutrophilen erhalten hat, vorzugsweise ausgewählt aus einem oder mehreren von Neutrophilen-reduzierender Apherese, Bestrahlungstherapie oder Verabreichung von Neutrophilen-reduzierenden Mitteln, insbesondere chemotherapeutischen Medikamenten, monoklonalen Antikörpern gegen Neutrophilenmarker, immunmodulierenden Medikamenten, Cytokinen oder Mischungen davon.

6. Verfahren nach einem der Ansprüche 1 bis 5, **dadurch gekennzeichnet, dass** das Individuum ein Individuum ist, das eine Behandlung zur Steigerung der Anzahl der Lymphozyten, vorzugsweise eine Verabreichung von IL-2 oder eine Verabreichung von Lymphozyten, erhalten hat.

7. Verfahren nach einem der Ansprüche 1 bis 6, **dadurch gekennzeichnet, dass** die Differenz der Anzahl der Neutrophilen und der Anzahl der Lymphozyten (Neutrophile minus Lymphozyten; NML) bestimmt wird und
- (b4) Identifizieren als ein Individuum mit guter Prognose für einen erhöhten klinischen Nutzen, wenn NML niedriger oder gleich einem NML-Grundlinien-Level von 2,4 bis 4,4 x 10⁹ Zellen pro Liter Blut, insbesondere niedriger oder gleich 3,4 x 10⁹ Zellen pro Liter Blut ist; oder
- (b5) Identifizieren als ein Individuum mit schlechter Prognose für einen erhöhten klinischen Nutzen, wenn NML höher als ein NML-Grundlinien-Level von 2,4 bis 4,4 x 10⁹ Zellen pro Liter Blut, insbesondere höher als 3,4 x 10⁹ Zellen pro Liter Blut ist.

## Revendications

1. Procédé pour identifier le pronostic d'un bénéfice clinique amélioré chez un individu souffrant d'une tumeur épithéliale, comprenant
- (a1) la détermination du nombre de lymphocytes dans un échantillon de sang dudit individu in vitro, et
- (a2) la détermination du nombre de cellules neutro philes dans un échantillon de sang dudit individu in vitro, et
- (b) l'identification de l'individu en tant qu'individu ayant un mauvais pronostic de bénéfice clinique amélioré, si le nombre de lymphocytes est inférieur ou égal à une valeur de nombre de lymphocytes de base de 1,4 à 1,8 x 10⁹ par litre de sang, notamment inférieur ou égal à 1,6 x 10⁹ par litre de sang et le nombre de cellules neutrophiles est supérieur à une valeur de nombre de cellules neutrophiles de base de 4,0 à 6,0 x 10⁹ par litre de sang, notamment supérieur à 5,0 x 10⁹ cellules neutrophiles par litre de sang.

2. Procédé suivant la revendication 1, **caractérisé en ce que** le bénéfice clinique amélioré est un temps total de survie amélioré.

3. Procédé suivant la revendication 1 ou 2, **caractérisé en ce que** ladite tumeur épithéliale est le cancer du sein, le cancer du poumon, notamment le cancer pulmonaire non à petites cellules (NSCLC), le cancer de l'estomac, le cancer du pancréas, le cancer de la prostate, le carcinome des ovaires ou le cancer colo-rectal, notamment le cancer colo-rectal.

4. Procédé suivant l'une quelconque des revendications 1 à 3, **caractérisé en ce que** ledit individu est un patient atteint d'une tumeur, au stade III ou IV de ladite tumeur, classée d'après l'American Joint Committee on Cancer Manual for Staging Cancer, 6ème Edition (2002), notamment un patient atteint d'une tumeur au stade IV.

5. Procédé suivant l'une quelconque des revendications 1 à 4, **caractérisé en ce que** ledit individu est un individu qui a reçu un traitement de réduction des cellules neutrophiles, choisi de préférence parmi un ou plusieurs des traitements consistant en une aphérèse de réduction des cellules neutrophiles, une radiothérapie ou l'administration d'agents réduisant les cellules neutrophiles, notamment d'agents chimio-thérapeutiques, d'anticorps monoclonaux contre des marqueurs de cellules neutrophiles, de médicaments immunomodulateurs, de cytokines ou de leurs mélanges.

6. Procédé suivant l'une quelconque des revendications 1 à 5, **caractérisé en ce que** ledit individu est un individu qui a reçu un traitement d'augmentation du nombre de lymphocytes, de préférence l'administration de IL-2 ou l'administration de lymphocytes.

7. Procédé suivant l'une quelconque des revendications 1 à 6, **caractérisé en ce que** la différence du nombre de cellules neutrophiles et du nombre de lymphocytes (cellules neutrophiles moins lymphocytes ; NML) est déterminée ; et
- (b4) l'individu est identifié en tant qu'individu présentant un bon pronostic de bénéfice clinique amélioré, si le NML est inférieur ou égal à une valeur de NML de base de 2,4 à 4,4 x 10⁹ cellules par litre de sang, notamment inférieur ou égal à 3,4 x 10⁹ cellules par litre de sang ; ou
- (b5) l'individu est identifié en tant qu'individu ayant un mauvais pronostic de bénéfice clinique amélioré, si Le NML est supérieur à une valeur de NML de base de 2,4 à 4,4 x 10⁹ cellules par litre de sang, notamment supérieur à 3,4 x 10⁹ cellules par litre de sang.
